# EUROPEAN PATENT APPLICATION

(11) **EP 1 538 163 A2**
(43) Date of publication of application: **08.06.2005**
(21) Application number: 04025899.8
(22) Date of filing: 01.11.2001
(51) Int. Cl.: C07K 14/47, C12N 15/63, C07K 16/18, A61K 38/17, A61K 38/57, G01N 33/68

(54) **Phosphorylated Amyloid-Beta 1-43 Protein and its use in the treatment of Alzheimer's disease**

(30) Priority: 01.11.2000 GB 0026738; 01.11.2000 GB 0026739
(62) Divisional of application: 01980678.5
(71) Applicant: Insight Biotechnology Limited, Wembley, Middlesex HA9 7YN (GB)
(72) Inventor: Milton, Nathaniel Gavin Nicholas, Dulwich London SE22 8QE (GB)
(74) Representative: Jappy, John William Graham

(57) **Abstract**

A phosphorylated Amyloid-β protein (residues 1-43) is identified and is used to prepare therapeutic agents that are useful in the treatment or prevention of Alzheimer's disease.

## Description

### Field of the Invention

This invention relates to peptides and drugs that target proteins implicated in the progression of Alzheimer's disease. The peptides are also highly specific targets for therapeutic reagents that are useful for detecting, preventing and treating Alzheimer's disease.

### Background of the Invention

Alzheimer's disease is a debilitating physical disease, responsible for just over half of the 670,000 cases of dementia in the UK. One of its proposed mechanisms of action is via an alteration in the structure of the Amyloid-β (Aβ) protein (Selkoe, Nature 399: A23-A31 (1999)).

The Aβ protein is generated from the Amyloid-β Precursor Protein (AβPP) with the major forms Aβ 1-42 and Aβ 1-40 and the N-terminally truncated P3 peptides (Aβ 17-40 and Aβ 17-42) being generated by alternative enzymatic processing of AβPP. The C-terminally extended forms of Aβ (Aβ 1-42 and Aβ 17-42) show increased ability to form fibrils and are thought to have a causative action in the neurodegeneration seen in Alzheimer's disease (Mattson, Physiol. Rev. 77:1081-1132 (1997); Rosenblum, J. Neuropath. Exp. Neurol. 58: 575-581 (1999)).

All the major forms of Aβ contain a functional neurotoxic domain (Aβ 25-35) and mediate their neurotoxicity by binding to the intracellular Aβ-binding protein ERAB, an alcohol dehydrogenase (Yan *et al*., J. Biol. Chem. 274: 2145-2156 (1999); Yanker *et al*., Science 250: 279-282 (1990)). The major forms of Aβ also inhibit hydrogen peroxide breakdown by the antioxidant enzyme catalase, an effect that involves a direct high affinity binding reaction (Milton, Biochem. J. 344: 293-296 (1999)).

The Aβ 31-35 peptide is the shortest cytotoxic form of Aβ, inhibits catalase and inhibits binding of Aβ 1-42 to catalase (Milton, Biochem. J. 344: 293-296 (1999)). Both catalase and antibodies specific to this region prevent Aβ cytotoxicity, suggesting that compounds which specifically bind Aβ 31-35 may be of therapeutic value in the treatment of Alzheimer's disease.

The Aβ 16-20 region has been shown to be responsible for binding to ERAB (Oppermann *et al*., FEBS Lett. 451: 238-242 (1999)). Antibodies which block Aβ binding to ERAB prevent Aβ 1-42 cytotoxicity, suggesting that compounds which specifically bind Aβ 16-20 may also antagonise actions of Aβ.

It has also been proposed that an alteration in the structure of the Aβ protein may be an important determinant of cytotoxicity (Selkoe, Nature 399: A23-A31 (1999)). Chronic inhibition of phosphatases can cause Alzheimer's-like pathology (Arendt *et al*., Neurobiol. Aging; 19:3-13 (1998)) suggesting that Alzheimer's pathology may be due to an imbalance of kinase/phosphatase levels. The appearance of Aβ plaques in such animal models suggests that phosphorylation actions are crucial in the biochemical processes underlying Aβ plaque formation. The ability of cyclin-dependent kinase inhibitors to prevent Aβ toxicity also suggests a key role for such kinases in the toxic actions of Aβ (Giovanni *et al*., J. Biol. Chem; 274:19011-6 (1999); Alvarez *et al*., FEBS Lett; 459: 421-6 (1999)). These enzymes specifically phosphorylate serine and threonine residues within substrates and play roles in cell division and apoptosis.

The cyclin-dependent kinase cdc2 phosphorylates the tau protein, which is a major component of the neurofibrillary tangles characteristic of Alzheimer's disease. The cdc2 kinase also phosphorylates the AβPP and this event is thought to modulate the processing events which lead to the production of the mature Aβ peptide forms. There are, however no known cdc2 recognition sites on the Aβ peptide itself.

Anti-sense peptide sequences are derived from the complementary strand of DNA encoding a given protein, read in the same open reading frame (ORF). They can also be derived directly from the amino acid sequence of a protein, via reverse translation to produce a complementary DNA sequence. However, due to the degeneracy of the genetic code, there is typically more than one anti-sense sequence for any one protein. The complementary DNA strand for each individual amino acid can be read in either the forward 3'-5' or reverse 5'-3' direction, adding further degeneracy to the potential anti-sense peptide sequences. Anti-sense peptides have been shown to bind with high affinity to the given protein due to hydropathic interactions. Anti-sense peptides have also been shown to have sequence similarity to receptor binding sites and compounds, such as antibodies, that specifically bind such anti-sense peptides, have been used to isolate receptors (Bost & Blalock, Methods Enzymol; 168: 16-28 (1989), the content of which is incorporated herein by reference).

### Summary of the Invention

The present invention is based on the surprising finding that Aβ anti-sense peptides (AβAS) have sequence similarity with components of the cyclin-dependent kinase enzyme complex and that Aβ is phosphorylated in the Alzheimer's brain. This suggests (for the first time) that there is a direct biochemical interaction between Aβ and cyclin-dependent kinase enzymes.

The present invention is also based on the discovery of amino acid sequence similarities between an AβAS peptide and specific regions in ERAB and catalase.

According to a first aspect of the invention, a peptide comprises the anti-sense sequence of Aβ 1-43, or a fragment thereof, capable of binding to the Aβ protein within the Aβ 1-43 region, or a homologue thereof with the same hydropathic profile, or at least 60% sequence similarity.

Peptides of the invention may be used to target the Aβ protein to prevent phosphorylation by a protein kinase, or to prevent binding to catalase. Alternatively, the peptides may be used in assays to identify therapeutic agents that are capable of preventing interactions between Aβ and a protein kinase, or which modify interactions between Aβ and catalase.

According to a second aspect of the invention, the peptides may be used in the manufacture of a medicament for therapy of a condition mediated by either phosphorylation of Aβ, and/or the binding of endogenous Aβ to catalase.

According to a third aspect of the invention, the peptides are used in an assay for the identification of an agent that either prevents phosphorylation of Aβ, and/or inhibits the binding of endogenous Aβ to catalase. The assay comprises contacting a target agent with a peptide of the invention and Aβ protein, and determining whether the agent prevents the peptide binding to Aβ protein, when compared against a control where no target agent is present.

The realisation that cdc2 enzyme can interact with a specific region of Aβ, resulting in Aβ phosphorylation, allows new treatments to be developed, to prevent phosphorylation, and to treat Alzheimers disease.

According to fourth aspect of the invention, a protein kinase inhibitor is used in the manufacture of a medicament for the treatment of Alzheimer's disease, the inhibitor being targeted to prevent phosphorylation of the Aβ protein, to exert its therapeutic effect.

The present invention may also be used in a diagnostic application.

According to a fifth aspect of the invention, a method for determining whether a patient is at risk from Alzheimer's disease, comprises analysing a patient sample that contains Aβ to determine whether any of the Aβ is phosphorylated, where the detection of phosphorylated Aβ indicates a risk of Alzheimer's disease.

The anti-sense peptides of the invention may also be used in a vaccine. Further, a phosphorylated Aβ fragment may be used, either to generate antibodies specific for the phosphorylated form, or as an antigen in a vaccine composition.

### Brief Description of the Drawings

The present invention is illustrated with reference to the following figures, wherein:
Figure 1 shows the AβAS forward (F) peptide sequences derived from the cDNA strand complementary to the coding strand, i.e. read in the 3'-5' direction, "*" refers to a stop codon and "Alt AA" refers to an alternative amino acid which may be used as a replacement due to degeneracy of the sequence in the coding (5' to 3') strand;
Figure 2 shows the AβAS reverse (R) sequences, where "Rev 3"' refers to the DNA of the complementary strand, read in the 5' to 3' direction for each amino acid coding triplet;
Figure 3 shows the AβAS consensus (C) sequence derived from a comparison of AβAS(F) and AβAS(R) sequences;
Figure 4 shows a comparison of an Aβ anti-sense amino acid sequence with the amino acid sequences of cyclin-dependent kinase enzymes;
Figure 5 shows the binding of biotinylated Aβ 1-40 to recombinant human cdc2 in the presence of Aβ fragments, the cdc2 substrate peptide CSH 103 and the AβAS(F) peptides 14-23 and 27-36;
Figure 6 shows the phosphorylation of biotinylated Aβ 1-42 (hatched columns), Aβ1-40 (open columns) and Aβ 25-35 (closed columns) by human cdc2/cyclin-B1 in the presence of Aβ 17-28, the cdc2 119 - 122 fragment (CDK1 P) and the purinergic cdc2 inhibitor olomoucine;
Figure 7 shows the effects of Aβ 17-35 (open circles), Aβ17-35 S26A derivative (open squares) and Aβ17-35 pS26 (closed squares) on the MTT reduction in human NT-2 neurons;
Figure 8 shows the levels of phosphorylated Aβ peptide measured in extracts of human NT-2 neurons after exposure to Aβ 17-35 derivatives in the presence (closed columns) or absence (open columns) of the cdc2 inhibitor olomoucine;
Figure 9 shows the binding of biotinylated Aβ 1-40 to recombinant human cyclin B1 in the presence of Aβ fragments, the cdc2 substrate peptide CSH 103 and the AβAS(F) peptides 14-23 and 27-36;
Figure 10 shows the effects of Aβ peptides and olomoucine on human cdc2/cyclin-B1 phosphorylation of the histone H1 peptide;
Figure 11 shows the effects of Aβ peptides alone (open columns) or in the presence of the AβAS(F) 14-23 peptide (closed columns) or AβAS(F) 27-36 peptide (hatched columns) on the viability of SP2/0-Ag-14 mouse myeloma cells; and
Figure 12 shows the effects of Aβ peptides alone (open columns) or in the presence of the AβAS(F) 14-23 peptide (closed columns) or AβAS(F) 27-36 peptide (hatched columns) on catalase enzyme activity.

### Description of the Invention

The present invention is based on an analysis of anti-sense peptides derived from Aβ, to identify proteins that interact with the Aβ protein. Comparing the anti-sense sequences with known proteins, to identity sequence homologies, identified potential binding sites on known proteins that interact with Aβ. This has resulted in the identification of the precise regions of cdc-2, Cyclin B1, ERAB and catalase that are involved in protein binding.

The term "anti-sense peptide" is used herein to define an amino acid sequence that corresponds to that derived from a DNA sequence complementary to the normal coding sequence. As is well known in the art, DNA usually exists as a duplex with one strand being the coding strand which is expressed in the 5' to 3' direction. The complementary strand is not normally expressed but acts as a template for RNA polymerase, and extends in the 3' to 5' direction. The sequence of the complementary strand can be used to derive the anti-sense peptide, either through the use of synthetic methods or by recombinant DNA technology.

The principle of anti-sense peptides is that the hydropathic character of a peptide derived from the coding strand will be opposite to that derived from the complementary strand. Therefore, even though the actual anti-sense amino acid sequence will be very different from that derived from the coding strand, there will be a relationship in respect of the hydropathic character. This is explained in Blalock and Smith, Biochem. Biophys. Res. Comm. 121(1): 203-207 (1984) and Blalock and Bost, Biochem. J., 234: 679-683 (1986), the content of each being incorporated herein by reference. Because an anti-sense peptide will, in general, have a hydropathy profile opposite to that of the corresponding sense. peptide, it is expected that both will undergo protein-protein interactions.

An anti-sense peptide of the invention will correspond to that derived from the complementary strand read in the 3' to 5' direction (see SEQ ID NO. 3). Further, an anti-sense peptide may also be derived by reversing the order of each trimer (amino acid encoding) DNA sequence of the complementary strand, to encode a different amino acid (see SEQ ID NO. 5). For example, if the complementary strand (3' to 5') is: 3'-AAT GAC-5' (SEQ ID NO. 11) then the reverse sequence for each trimer is:

3'-TAA CAG-5' (SEQ ID NO. 12)

Peptides of the invention derived in this way have similar hydropathy profiles and can bind to the Aβ 1-43 region.

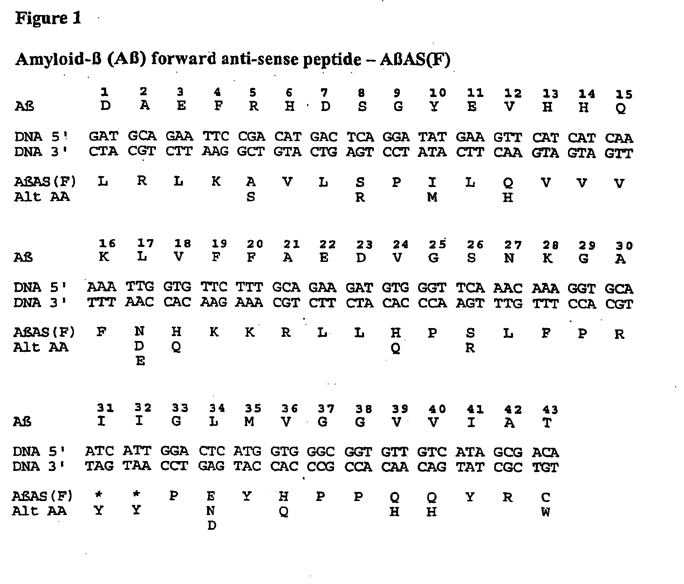

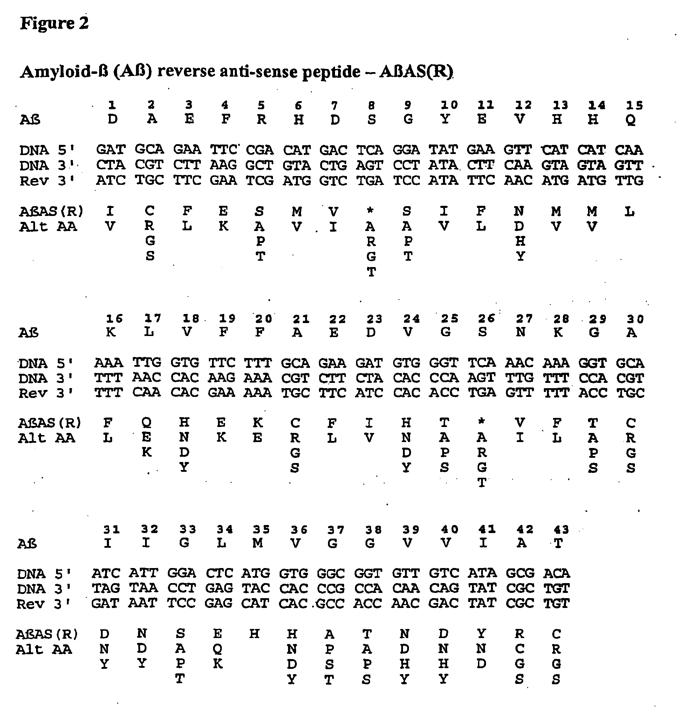

The sequence of an anti-sense peptide may vary due to the degeneracy of the coding strand. For example, the amino acid valine is encoded by GTG or GTT. The complementary strand will therefore be either CAC or CAA encoding histidine or glutamine, respectively. This is also shown in Figures 1 and 2 for the alternative anti-sense sequences derived from Aβ 1-43.

The sequence of the complementary strand may encode a stop codon. In these circumstances, it is necessary to introduce an appropriate amino acid residue. The replacement amino acid residue will usually be derived from an alternative coding sequence for the amino acid of the coding strand. For example, if the coding strand is ATC (isoleucine), the complementary strand is a stop codon TAG. Isoleucine is also encoded by ATA, the complement of which encodes tyrosine (TAT). Therefore, tyrosine is used at the position corresponding to the stop codon. This is shown in Bost & Blalock, Methods Enzymol; 168: 16-28 (1989), the content of which is incorporated herein by reference.

For the avoidance of doubt, reference to the Aβ 1-43 region means the amino acid numbering for the conventional Aβ protein, shown as SEQ ID NO. 2.

Functional fragments thereof, i.e. smaller peptides that retain the ability to bind to the Aβ 1-43 region, are also within the scope of the invention. The fragments will usually be at least 6 amino acids in length, typically the fragments will be at least 8 amino acids in length. In preferred embodiments, the fragments comprise the anti-sense derivatives of Aβ 12-24 or Aβ 31-35. In further preferred embodiments, the fragments are the anti-sense derivatives of Aβ 3-30, Aβ 17-35, Aβ 17-24, Aβ 12-28, Aβ 14-35 or Aβ 25-35.

The binding of Aβ to itself can occur in both parallel and anti-parallel orientations (Serpell, Biochimica et Biophysica Acta 1502: 16-30 (2000)) with consequent interactions between for example two N-terminals in parallel binding or an N and a C terminus in anti-parallel binding. If binding of a peptide to an anti-sense peptide sequence were to occur in an anti-parallel orientation then the anti-sense peptide would have to be synthesized in the anti-parallel direction with the C terminus occupying the N-terminus of the resultant peptide. Similarly an anti-parallel binding interaction between a binding protein and a peptide may be identified by comparison of the anti-sense peptide sequence in the C-terminus to N-terminus orientation with the binding protein sequence in the normal N-terminus to C-terminus orientation.

The anti-sense peptides of the invention can therefore have the given sequence in either the N-terminus to C-terminus orientation, or the C-terminus to N-terminus orientation. This is demonstrated by SEQ ID NO. 4 (N-terminal amino acid first) and SEQ ID NO. 7 (C-terminal amino acid first).

The binding of a peptide (or fragment) to the endogenous Aβ 1-43 region may be determined as shown in the Examples, and in Milton, Biochem. J. 344: 293-296 (1999).

The peptides bind with a dissociation constant (Kd) of less than 50 µM, preferably less than 10 µM.

The term "homologue" is used herein in two separate contexts. The first is to refer to peptide sequences that share the same hydropathy profile as the peptides of the invention. This may be determined by analysing the peptide sequence and evaluating what alternative amino acids could be used as a replacement based on hydropathic character. Table 1 groups together those amino acids with a similar hydropathic character and which can be substituted for an amino acid specified in the anti-sense peptide sequence.

**Table 1**

| **Amino acid** | **Acceptable substitutions** |
|---|---|
| Alanine (Ala) | Arg, Gly, Pro, Ser, Thr |
| Arginine (Arg) | Cys, Gly, Ser, Thr, Trp |
| Asparagine (Asn) | Asp, Gln, Glu,, His, Lys; Tyr |
| Aspartic acid (Asp) | Asn, Gln, Glu,, His, Lys, Tyr |
| Cysteine (Cys) | Arg, Gly, Ser, Trp |
| Glutamic Acid (Glu) | Asn, Asp, Gln, Lys, |
| Glutamine (Gln) | Asn, Asp, Glu, His, Lys, Tyr |
| Glycine (Gly) | Ala, Arg, Cys, Ser, Thr, Trp, |
| Histidine (His) | Asn, Asp, Gln, Tyr |
| Isoleucine (Ile) | Leu, Met, Val |
| Leucine (Leu) | Ile, Phe, Val |
| Lysine (Lys) | Asn, Asp, Gln, Glu |
| Methionine (Met) | Ile, Val |
| Phenylalanine (Phe) | Leu, |
| Proline (Pro) | Ala, Ser, Thr |
| Serine (Ser) | Ala, Arg, Cys, Gly, Pro, Thr, Trp |
| Threonine (Thr) | Ala, Arg, Gly, Pro, Ser |
| Tryptophan (Trp) | Arg, Cys, Gly, Ser |
| Tyrosine (Tyr) | Asn, Asp, Gln, His |
| Valine (Val) | Ile, Leu, Met |

The term "homologue" is also used to refer to peptides that share levels of sequence identity or similarity. Levels of identity or similarity between amino acid sequences can be calculated using known methods. Publicly available computer based methods include BLASTP, BLASTN and FASTA (Atschul *et al*., Nucleic Acids Res., 25:3389-3402 (1997)), the BLASTX program available from NCBI, and the GAP program from Genetics Computer Group, Madison WI.

The levels of identity and similarity referred to herein are based on the use of the BLASTP program. All BLAST searches were carried out using the Standard protein-protein BLAST (blastp) on the NCBI web site (www.ncbi.nlm.nih.gov/BLAST) with the BLOSUM62 matrix and Gap Costs of 11 for Existence and 1 for Extension. The statistical significance threshold for reporting matches against database sequences (E) was reset to 100 to account for the use of short peptide sequences in the search. For BLAST comparisons between AβAS peptides and already identified Aβ binding proteins, the same parameters were used except that the E value was reset to 100000 to ensure identification of all potential regions of similarity. Alignments of AβAS fragments of <5 amino acids were considered non-significant under these conditions. Sequences containing significant gaps (> 10%) were not used since hydropathic binding interactions require a direct alignment of each Aβ residue with its complementary anti-sense peptide or binding domain residue.

It is preferable if there is at least 60% sequence identity or similarity to the specified peptides, preferably 70%, more preferably 80% and most preferably greater than 90%, e.g. at least 95%. The peptides should retain the ability to bind to the Aβ protein.

Synthetic amino acid derivatives may also be used. For example, the shifting of substitutents within an amino acid residue, from a C atom to a N atom, to produce a peptide having greater resistance to proteolysis, and other modifications, are known and are included within the scope of this invention.

Peptides of the invention may be synthesised using conventional methods known in the art and can be obtained to order from commercial sources. Peptide synthesis methods are also disclosed in Chan & White, Fmoc Solid Phase Peptide Synthesis: A Practical Approach (2000).

Alternatively, the peptides may be produced using recombinant DNA technology that ensures that the anti-sense (complementary) DNA sequence is expressed. This can be accomplished using techniques known to those skilled in the art. For example, the DNA sequence to be expressed can be inserted into an appropriate expression vector that contains the necessary regulatory apparatus, e.g. promoters, enhancers etc, to enable expression to occur. The DNA sequence will be in the 5' to 3' direction, for expression, but will have the same nucleotide sequence as that given for the complementary (3' to 5') strand. The DNA sequence may therefore be a synthetic polynucleotide. The expression vector can then be inserted into an.appropriate host cell, to enable expression to occur. Suitable methods are disclosed in Sambrook *et al*, Molecular Cloning, A Laboratory. Manual (1989), and Ausubel *et al*, Current Protocols in Molecular Biology (1995), John Wiley & Sons, Inc., the content of each being incorporated herein by reference.

As stated previously, the present invention is based on the finding that the Aβ protein, within the region 1-43, contains the sites that interact with other proteins, and that these protein-protein interactions may be implicated in Alzheimer's disease. The DNA sequence that encodes the Aβ 1-43 region is shown as SEQ ID NO. 1 with the encoded amino acid sequence shown as SEQ ID NO. 2. The complementary DNA sequence is also shown (SEQ ID NO. 3). The anti-sense sequence is shown as SEQ ID NO. 4. The reverse anti-sense DNA sequence is shown as SEQ ID NO. 5, with its encoded product shown as SEQ ID NO. 6.

The peptide in the C-terminus to N-terminus orientation to that of SEQ ID NO. 4, is shown as SEQ ID NO. 7, and that in the C-terminus to N-terminus orientation to that of SEQ ID NO. 6 is shown as SEQ ID NO. 8.

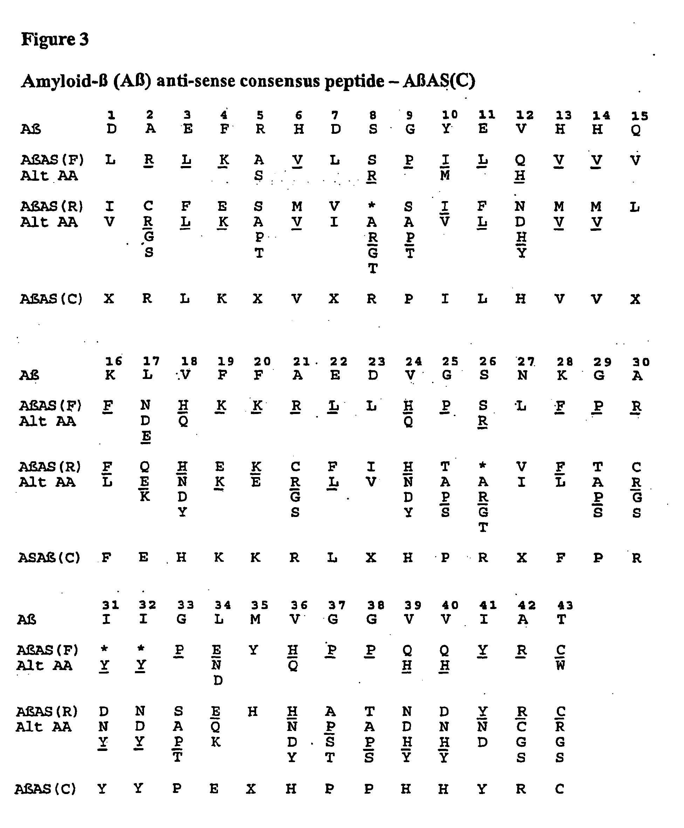

A further AβAS sequence (SEQ ID NO. 9) was derived by use of the consensus amino acid sequences that are found at the same position of each form of anti-sense peptide, i.e. those in the forward or reverse orientation etc. This is most clearly shown in Figure 3, where a sequence alignment of the various forms of the anti-sense peptides shows which amino acids are common to each position (AβAS(C)). This.is also explained in Bost & Blalock, Methods Enzymol; 168: 16-28 (1989). The peptide in the C-terminus to N-terminus orientation to that of SEQ ID NO. 9, is shown as SEQ ID NO. 10.

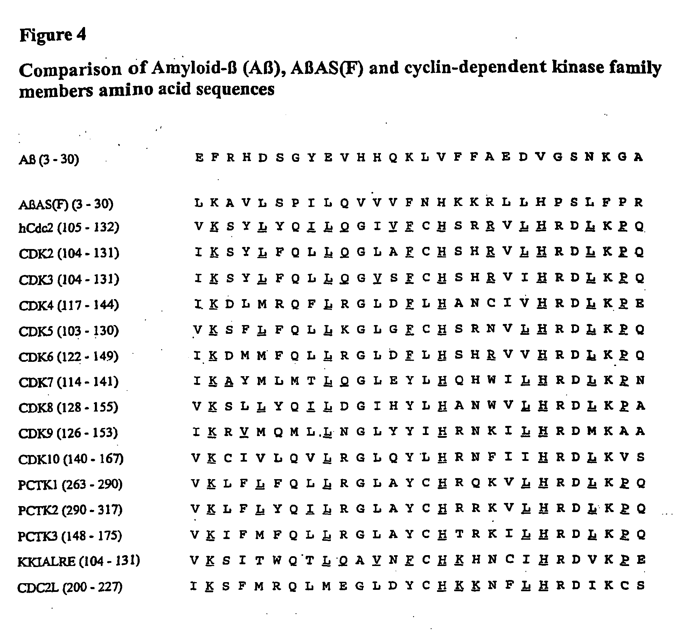

The investigations disclosed herein demonstrate that Aβ binds to and is phosphorylated by the human cdc2 protein kinase. The cdc2 kinase is a member of the cyclin-dependent kinase (CDK) family and the structural features of CDK substrates have been characterised. These features include the presence of β-turn regions containing the target serine or threonine residue. The serine 26 residue in Aβ is located within a β-turn region and this structural feature may be important for the Aβ phosphorylation reaction. The Aβ sequence, however, does not contain the cdc2 substrate consensus sequence and it is therefore likely that the enzyme-substrate complex formation between Aβ and cdc2 is mediated via a novel mechanism. Anti-sense peptides are known to bind peptides via hydropathic interactions and such binding between cdc2 and Aβ with an alignment of the active site of cdc2 with Aβ serine 26 provides a mechanism for the observed Aβ phosphorylation reaction. Since the CDK family of kinases share similar structural features around the ATP binding and phosphate group transfer residues, it is possible that Aβ could be phosphorylated by other CDK kinases, and this may explain why different groups have shown roles for different CDK enzymes in Aβ cytotoxicity. The sequence similarities of CDK family members around the cdc2 active site is illustrated in Figure 4.

Therefore, molecules which specifically prevent the phosphorylation of Aβ may be of therapeutic use. Anti-sense peptides of at least 6 amino acids, either alone or chemically linked to other protein kinase inhibitor molecules, derived from the Aβ 1-43 sequence, may act as inhibitors of Aβ phosphorylation and be useful in the treatment of Alzheimer's disease.

Fragments of Aβ may also be useful as antagonists, to inhibit the phosphorylation of the endogenous Aβ. In this embodiment, it is desirable to administer fragments that are capable of preventing phosphorylation, but which are also non-cytotoxic. It may therefore be desirable to administer fragments that do not contain the cytotoxic portion 31-35, or which are modified at one or more of these amino acid sites.

Compounds that bind specifically to phosphorylated Aβ may also be useful in the diagnosis of Alzheimer's disease. Novel antibodies may be raised using known antibody production techniques. For example, a peptide of the present invention, acting as an antigen, may be administered to an animal to produce an antibody-rich serum. This "antiserum" can be purified, to remove unwanted antibody molecules, by, for example, affinity fractionation using phosphorylated Aβ. Monoclonal antibodies may also be raised by, for example, animal or *in vitro* immunisation techniques and fusion of antigen-exposed spleen cells to a myeloma cell line to produce hybridoma cell lines that secrete antibody. By screening hybridoma cell lines with a peptide of the invention, specific antibody-producing cell lines may be established.

In a preferred embodiment, a peptide fragment of the natural Aβ protein in the phosphorylated state, is used to raise antibodies that are specific for phosphorylated Aβ, and not for the non-phosphorylated form. The techniques of phage display or ribosome display, both of which are conventional in the art, may be used to select those antibodies with high affinity, preferably greater than 10⁻³ M, more preferably greater than 10⁻⁵ or 10⁻⁶ M. The antibodies may be useful in therapy or diagnostic assays.

A previous study has shown that immunisation with Aβ prevents Alzheimer's-like pathology in an animal model (Schenk *et al*., Nature; 400: 173-177 (1999)). The use of a phosphorylated Aβ derivative may direct the body's immune system against a more cytotoxic form of Aβ and hence may be a more suitable immunogen for such treatment. Thus, immunization with a phosphorylated Aβ fragment may be used as a treatment for Alzheimer's disease and as a preventative medicine.

It may also be desirable to administer an antigenic fragment of the protein kinase, e.g. cdc2, or cyclin which may also act as a preventative medicine. Antibodies raised against the protein kinase or cyclin may also be of therapeutic or diagnostic use. It is preferable to use at least that part of the protein kinase or cyclin that contains the region associated with Aβ binding, as the antigenic fragment.

The immunogen may be administered via any suitable route, preferably intravenously. Suitable pharmaceutically-acceptable diluents and carriers will be known to those skilled in the art. Adjuvants may also be administered, e.g. Alum, as is known in the art. A suitable amount of the therapeutic to be administered, can be arrived at by the skilled person based on conventional formulation technology.

If the natural Aβ protein (or fragments thereof), is to be used as an antigenic component of a vaccine composition, either in the phosphorylated state or non-phosphorylated state, it is desirable to ensure that the 31-35 region is deleted or modified to ensure that the Aβ antigen is not cytotoxic.

In a further embodiment, if a Aβ peptide is to be administered as an antigen in the non-phosphorylated form, it may be desirable to modify the peptide to replace the amino acid residue susceptible to phosphorylation, to ensure that no phosphorylation occurs.

Peptides, antibodies and compositions of the present invention may be useful in a method of treating or diagnosing Alzheimer's disease.

For example, a sample from a patient (blood sample, tissue sample etc.) that contains Aβ can be used to detect whether phosphorylated Aβ is present. The phosphorylated Aβ can be detected, for example, by the use of an antibody that has specificity for phosphorylated Aβ and no or reduced specificity for non-phosphorylated Aβ. Alternatively, levels of phosphorylated Aβ can be determined by measuring the cytotoxicity of the Aβ sample, compared to a non-phosphorylated Aβ sample.

The peptides of the invention may be used in assays to identify therapeutic molecules that can prevent phosphorylation of Aβ from occurring. For example, combinatorial chemistry could be used to develop target therapeutic molecules, which are then screened for activity. The target molecules can be brought into contact with Aβ protein, or a fragment thereof comprising the phosphorylation site of Aβ, and a protein kinase, e.g. p34-cdc2. If the presence of the target molecule results in reduced phosphorylation, then it may be a potential therapeutic candidate. Alternatively, the target molecule can be brought into contact with Aβ protein and an anti-sense peptide of the invention, and the efficacy of the target molecule determined on the basis of a reduction in binding affinity between the Aβ protein and the anti-sense peptide.

Preferably, the target molecule will be a protein kinase inhibitor that acts specifically at the Aβ target site. It is therefore preferable for the target molecule to have affinity for Aβ. Alternatively, a protein kinase inhibitor could be adapted to include a targeting molecule that has affinity for Aβ.

In an alternative embodiment, it may be useful to identify compounds that phosphorylate Aβ. Assays to identify phosphorylating compounds can be. designed so that Aβ (or a suitable fragment thereof) is brought into contact with the compound to be tested, in the presence of suitable reagents necessary to allow a phosphorylation reaction to proceed. The extent (if any) of phosphorylation can then be determined.

In a further .embodiment, the phosphorylated Aβ may be used as a target for compounds that inhibit or modify the biological action of the phosphorylated Aβ. Assays can be carried out to determine whether a target compound interacts selectively with the phosphorylated form of Aβ and alters the Aβ cytotoxicity.

In addition, the peptides of the invention can prevent Aβ binding to catalase, and may be useful in therapy or in assays to identify agents that prevent binding of Aβ to catalase.

The invention will now be further described by the following Examples with reference to the accompanying Figures.

### Example 1

To identify potential Aβ binding domains within human proteins, an anti-sense peptide approach was used. The forward Aβ anti-sense peptide (AβAS(F)) 1-43 (SEQ ID NO. 4) was derived by reading the complementary (non-coding) strand of DNA from the region encoding the Aβ 1-43 peptide in the 3'-5' direction; where the DNA encoded a stop codon, the nearest suitable replacement amino acid was substituted. The AβAS(F) sequence was used in a BLAST search to identify proteins with sequence similarity. Results showed a region of sequence similarity with the AβAS(F) 3-30 sequence having 46% identity and 68% similarity with the human cdc2 105-132 region (SEQ ID NO. 15). This indicated that Aβ 1-43 may be phosphorylated by cdc-2. The BLAST comparison between AβAS and human cdc2 (Accession No. GI 87058) also identified three other regions of sequence similarity. Cdc2 residues 56 to 63 (SEQ ID NO. 13) showing 50% identity and 75% similarity with AβAS 20-27; cdc2 residues 95 to 99 (SEQ ID NO. 14) showing 80% identity with AβAS 37-41; and cdc2 residues 229 to 238 (SEQ ID NO. 16) showing 40% identity and 50% similarity with AβAS 33-42.

Experiments were carried out to determine whether Aβ 1-43 binds to and is phosphorylated by cdc-2. It was found that Aβ 1-43 binds to and is phosphorylated by cdc-2 and that phosphorylation follows similar kinetics to known p34-cdc2 substrates and can be inhibited by chemicals and peptides known to inhibit p34-cdc2 kinases.

Biotinylated Aβ 1-42, Aβ 1-40 and Aβ 25-35 were prepared using a LinKit-Biolink kit (ISL, Paignton, UK). ELISA plates were coated with recombinant human cdc2 or the cdc2 119-133 peptide fragment (CDKP1) (1 µg ml⁻¹) in carbonate buffer and unoccupied sites blocked with 5% (w/v) dried milk. Biotinylated peptides (200 pM) were incubated alone, with control peptides (somatostatin) or with unlabelled Aβ peptides in PBS (containing 0.1% BSA and 0.05% Tween-20) at 37°C for 4 hours. After washing to remove unbound material, an alkaline phosphatase polymer-streptavidin conjugate (Sigma, Dorset, UK) was added and incubated at 37°C for 2 hours. After washing to remove unbound material p-nitrophenylphosphate substrate was added and absorbance at 405 nm determined. Affinity constants were determined by incubating cdc2 coated plates with biotinylated peptides (200 pM) plus Aβ peptides over a range of concentrations (0 - 10 µM) and detection of bound peptides was carried out by ELISA.

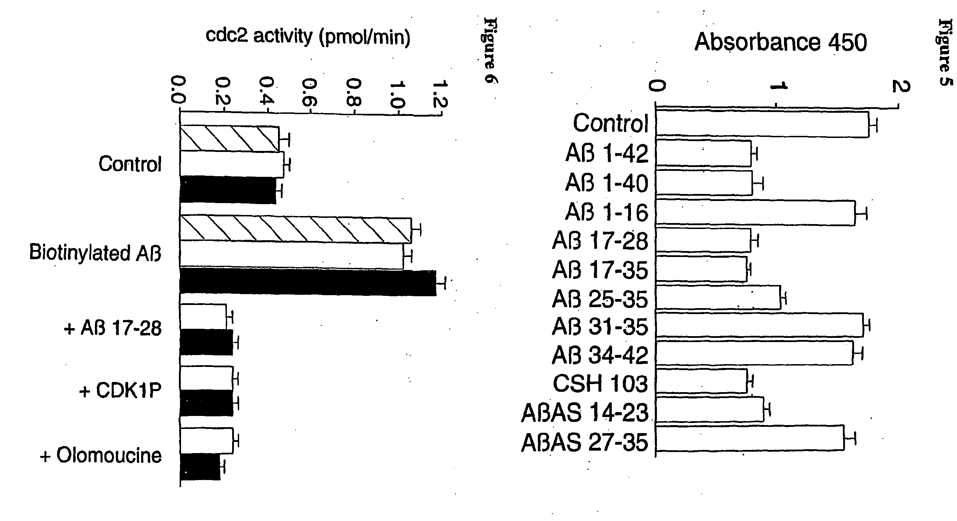

The results showed that Aβ 1-42, Aβ 1-40 and Aβ 25-35 bound to human cdc2 (Figure 5). The cdc2 substrate peptide CSH-103 (Sigma) and peptides containing Aβ residues 17-28 could inhibit the binding of Aβ to cdc2. The AβAS(F) 14-23 but not the AβAS(F) 27-36 peptide also inhibited binding. The binding of Aβ 1-40 was concentration dependent and showed an affinity constant (K_{D}) of 12.7 ± 4.3 µM. The biotinylated Aβ peptides also bound specifically to a synthetic peptide (CDKP1) corresponding to cdc2 residues 119-133. Binding of Aβ 25-35 to the CDKP1 peptide could be inhibited by peptides containing the Aβ 17-28 sequence and by either anti-Aβ 17-28 or anti-CDKP1 antibodies. The binding of Aβ to cdc2 was inhibited by the Aβ 17-28 but not the Aβ 31-35 fragments indicating that the cdc2 56-63 may also contribute to Aβ binding. The alignment within this cdc2 region of Aβ residue 23 (a negatively charged Aspartic acid residue) with the positively charged Arginine 59 of cdc2 suggested that a charge-based interaction may occur at this location. The tertiary structure of cdc2 suggests that the 56-63 region could play a role in interactions with the substrate bound to the active site region surrounding cdc2 residue 128. These observations suggest that this Aβ binding region may be an alternative target for therapeutic agents that would specifically disrupt Aβ phosphorylation.

Using the NetPhos 2.0 computer program, which predicts phosphorylation sites in proteins (Blom *et al*., J. Mol. Biol. 294: 1351-1362 (1999)) it was found that Aβ 1-42 contains three potential sites (serine 8, tyrosine 10 and serine 26). The NetPhos 2.0 scores were obtained from the output score of the ensemble of neural networks trained on that acceptor residue type and a value > 0.5 was considered significant. The scores for Aβ serine 8, tyrosine 10 and serine 26 were 0.963, 0.870 and 0.787 respectively. The alignment of the AβAS 26 residue, which is complementary to the Aβ serine 26 residue, with the proposed aspartic acid 128 active site residue of cdc2 that is involved in the transfer of the phosphate group from ATP to the substrate (Figure 4) suggests that cdc2 could phosphorylate Aβ.

For p34-cdc2 activity measurements, recombinant p34-cdc2/cyclin B1 (Promega, UK) was used. The activity of p34-cdc2 incubated with biotinylated Aβ 1-42, 1-40 and 25-35 was determined. Recombinant p34-cdc2 with an activity of 1U (incorporation of 1 pmol ATP/min/µg protein into a peptide substrate of the histone H1 sequence PKTPKKAKKL (SEQ ID NO. 22) was incubated with 25 µM biotinylated peptides in assay buffer (50 mM TRIS-HCl, 10 mM MgCl₂, 1 mM EGTA, 2 mM DTT, 40 mM β-glycerophosphate, 20 mM p-nitrophenylphosphate, 0.1 mM sodium vanadate, 50 µM ATP pH 7.4) plus test substances and [γ³²P]-ATP (specific activity 3,000 Ci/mmol) in a final volume of 25 µl. After incubation for 10 min at 30°C termination buffer (12.5 µl; 7.5 M guanidine HCl) was added. A 15 µl aliquot of each sample was spotted onto a streptavidin membrane (Promega, UK) to isolate the biotinylated peptides. The membrane was washed four times in 2 M NaCl, followed by four times in 2 M NaCl containing 1% (v/v) H₃PO₄ and finally twice in deionized H₂O to remove unbound material. The radioactivity of [γ³²]-ATP incorporated into the biotinylated peptides was measured by scintillation counting and the enzyme activity determined.

Results showed that Aβ 1-42, Aβ 1-40 and Aβ 25-35 incorporated ³²P from γ³²P-ATP (Figure 6) and that cdc2 caused the appearance of phosphorylated serine residues in Aβ 1-42, Aβ 1-40 and Aβ 25-35. Phosphorylation of Aβ was inhibited by olomoucine, a purinergic.cdc2 inhibitor, the CDKP1 peptide, Aβ 12-28 and Aβ 17-28. Kinetic analysis of the reaction showed that the phosphorylation was concentration dependent and the Michaelis constant (K_{M}) for the phosphorylation of Aβ 1-40 was 5.2 µM, which compared with a K_{M} of 2.7 µM for the H1 peptide substrate.

In order to assess the effects of Aβ phosphorylation on the cytotoxic properties of Aβ peptides, a series of Aβ 17-35 derivatives were synthesised. The 17-35 region of Aβ contains the serine residue (serine 26) which is proposed to be phosphorylated by p34-cdc2, and also contains the ERAB binding (17-20) and cytotoxic domains (31-35) thought to play roles in Aβ cytotoxicity. The peptides were tested in a cytotoxicity assay as follows. Human NT-2 (NTera2/D1) precursor cells were propagated in DMEM/F12 medium supplemented with retinoic acid for 5-6 weeks prior to harvesting and replating in the presence of mitotic inhibitors, to generate post-mitotic Human NT-2 neurons. For cytotoxicity experiments, 5 x 10³ cells/1 00 µl medium were plated in Poly-D-lysine coated 96 well plates. Test peptides (20 µM) were added directly to culture medium prior to incubation for 24 h. Cell viability was determined by measurement of MTT (3-(4,5-dimethylthiazol-2-yl)-2,5-diphenyl tetrazolium bromide) reduction (Shearman, Methods Enzymol. 309: 716-723 (1999)). After incubation with peptides MTT (10 µl: 12mM stock) was added and cells incubated for a further 4 hours. Cell lysis buffer [100 µl/well; 20% (v/v) SDS, 50 % (v/v) N,N-dimethylformamide, pH 4.7] was added and, after repeated pipetting to lyse cells, colorimetric determination of MTT formazan product formation was determined by measuring the absorbance change at 540 nm. Control levels in the absence of peptide were taken as 100%, with the. absorbance in the absence of cells taken as 0%.

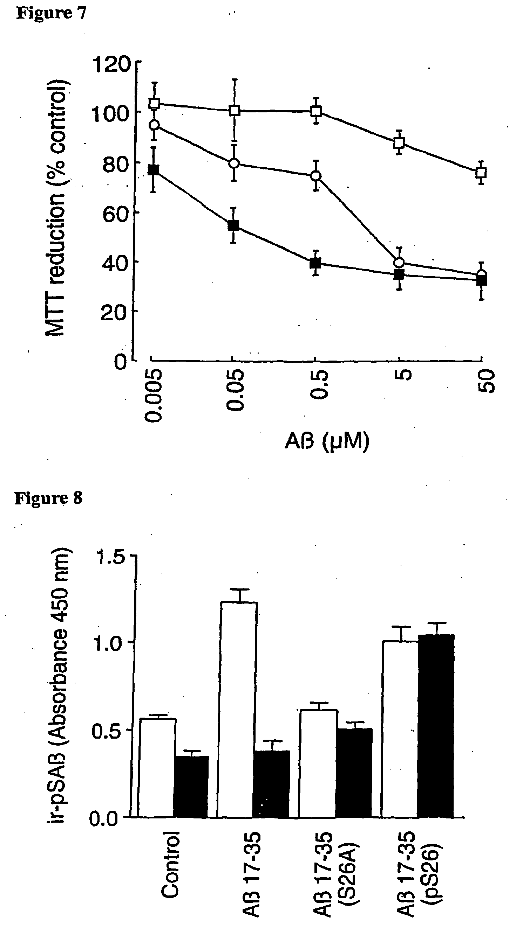

The Aβ 17-35 peptide caused a dose dependent reduction in MTT utilisation (Figure 7). The Aβ 17-35 S26A mutated peptide, in which the serine residue for phosphorylation has been mutated to an alanine residue, had no effect on MTT utilisation indicating that this mutation abolishes the cytotoxic potential of the peptide. An Aβ 17-35 peptide with a phosphorylated serine residue (Aβ 17-35 (pS26)) caused a dose dependent reduction in MTT utilisation and was significantly more potent than the non-phosphorylated peptide. These results suggest that the serine phosphorylation by p34-cdc2 or other kinases could be a key step in the cytotoxic actions of Aβ peptides.

To establish whether Aβ is phosphorylated in the Alzheimer's brain, Alzheimer disease brain sections were obtained from Novagen Inc (Madison, WI, USA. Cat No: 70298-3; Lot No: A301036). Sections were deparaffinised and extracted in DEA buffer supplemented with 0.1mM sodium vanadate. Extracts from NT-2 neurons were also prepared using the same buffer. Using a polyclonal anti-Aβ 15-30 antiserum plus Protein-A agarose, the Aβ was immunoprecipitated. The resultant extracts were further purified using a Sep-Pak C₁₈ extraction step. Columns were pre-wetted with methanol and 0.5M acetic acid and the samples were applied in 20% acetonitrile in 0.1% TFA. The columns were washed with 20% acetonitrile in 0.1% TFA prior to elution of bound peptide with 70% acetonitrile. After drying under a stream of nitrogen, samples were resuspended in appropriate buffer. For SDS-PAGE analysis, samples were resuspended in gel loading buffer and run on a 15% acrylamide gel. After immunoblotting onto nitrocellulose membranes, the blots were stained with anti-Aβ, anti-phosphoserine, anti-phosphotyrosine or anti-phosphothreonine antibodies. Bands were visualized with anti-mouse or anti-rabbit IgG-HRP conjugates and TMB membrane substrate.

SDS-PAGE analysis showed the presence of a phosphoserine containing band of a similar size to Aβ which could also be stained with a specific anti-Aβ monoclonal antibody (6F3D). The staining of this band with an anti-phosphoserine. antibody, but not the anti-Aβ antibody, was prevented by pretreatment of the extracts with alkaline phosphatase. No bands were stained using anti-phosphothreonine and anti-phosphotyrosine specific antibodies, confirming that the Aβ was only phosphorylated at one of its serine residues.

A specific immunoassay was used to measure Aβ phosphorylated on a serine residue (pSAβ) in cell extracts. ELISA plates were coated with anti-phosphoserine antiserum for pSAβ determination and blocked with 5% dried milk. Samples or synthetic Aβ standards (Aβ 17-35 pS) were applied in PBS containing 0.1% BSA plus 0.05%.Tween 20. Monoclonal antibody ALI-01 was added and incubated for 2 hrs. After washing to remove unbound material, ir-pSAβ was detected using an anti-rabbit IgG-HRP conjugate and TMB substrate. A similar assay in which the pS antibody was replaced with a polyclonal anti-Aβ antibody was used to measure Aβ levels.

Extracts from NT-2 neurons contained 3.16 ± 0.48 nmol/g Aβ of which 1.30 ± 0.05 nmol/g (41.1 ± 1.6%) was of the pSAβ form. Alzheimer's disease brain extracts contained 59.8 ± 3.8 nmol/g Aβ of which 12.6 ± 6.6 nmol/g (20.8 ± 10.7%) was of the pSAβ form. Human NT-2 neurons exposed to the Aβ 17-35, Aβ 17-35 pS26 and an S26A mutated Aβ 17-35 derivative showed increased levels of immunoreactive Aβ (ir-Aβ). Measurement of ir-pSAβ in the same cell extracts showed that cells exposed to Aβ 17-35 contained increased amounts of ir-pSAβ (Figure 8), whilst cells exposed to the Aβ 17-35 pS26 or Aβ 17-35 S26A peptides showed no difference to control cells. The increase in ir-pSAβ levels, but not ir-Aβ, when cells were treated with Aβ was prevented by the cdc2 inhibitor olomoucine.

Cyclins are co-factors for cdc2 which are required for enzyme activity. These proteins also contain substrate recognition sequences which may play a role in the recruitment of substrate molecules to the active cdc2/cyclin complex. The recombinant cdc2/cyclin enzyme complex used in the above phosphorylation experiments contained cyclin B1. To test if this protein contained an Aβ binding site the AβAS (R) reverse peptide sequence, read in the C to N direction (SEQ ID NO. 8) was used in a BLAST comparison with the cyclin B1 (GI 116176) protein sequence. Results showed a region with 30% sequence identity and 43% sequence similarity between this AβAS peptide and the cyclin B1 257-285 (SEQ ID NO. 17) region. Binding assays as described above using cyclin B1 coated plates were carried out.

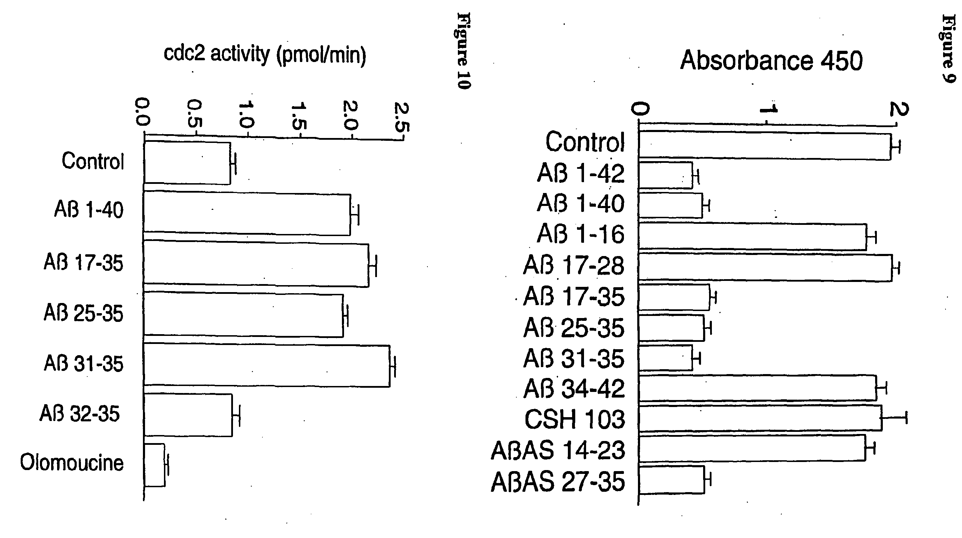

Results showed that cyclin B1 bound to biotinylated Aβ 1-40 and 25-35 (Figure 9). The binding was inhibited by Aβ 31-35 containing peptides. The affinity constant for Aβ 1-40 binding to cyclin B1 was 2.3 ± 0.5 µM. The binding could be inhibited by the forward AβAS(F) 27-36 but not the AβAS(F) 14-23 peptide.

Since Aβ binds to both the cdc2 and cyclin B1 components of the active enzyme it is possible that Aβ modulates the activity of the kinase. This was tested by performing kinase activity measurements using a biotinylated Histone H1 substrate peptide (PKTPKKAKKL) and measurement of incorporation of ³²P from ³²P-ATP as above. Results showed that Aβ 1-40, 17-35, 25-35 and 31-35 all increased the phosphorylation of the H1 peptide by cdc2/cyclin B1 (Figure 10), suggesting that Aβ could activate the kinase. The fragments capable of activation were the same as those which inhibited Aβ 1-40 binding to cyclin B1 and these results suggest that the binding to cyclin B1 may be a mechanism for the enzyme activation.

### Example 2

This Example shows the protein-protein interaction between the peptides of the invention and utility of the peptides of the invention as inhibitors of binding between Aβ and catalase.

The BLAST comparison between the AβAS(F) 1-43 peptide (SEQ ID 4) and human catalase (GI: 14763736) identified three regions of sequence similarity. Catalase residues 402 to 414 (SEQ ID NO. 20) showing 46% identity and 61 % similarity with AβAS 29-40; catalase residues 158 to 164 (SEQ ID NO. 18) showing 57% identity and 71% similarity with AβAS 25-31; and catalase residues 281 to 287 (SEQ ID NO. 19) showing 57% identity and 85% similarity with AβAS 11-17. The Aβ 31-35 fragment of Aβ binds to catalase (Milton 1999) and this suggests that the 402-414 region of catalase may contain the binding site. The presence of a gap in the alignment corresponding to catalase 407 which was inserted between Aβ residues 33 and 34 suggests that the 402-406 region of catalase may be of more importance. This is in agreement with the study of Milton *et al*., NeuroReport:12, 2561-2566 (2001) which identified these residues as the binding site.

The BLAST comparison between AβAS(F) 1-43 peptide (SEQ ID NO. 4) and human ERAS (GI: 2492759) identified a single region of sequence similarity. ERAB residues 101 to 109 (SEQ ID NO. 21) showing 44% identity and 55% similarity with AβAS 16-24. The Aβ 16-20 fragment of Aβ binds to ERAB (Oppermann, *et al*., FEBS Lett. 451: 238-242 (1999)) suggesting that the 101-109 region contains the ERAB binding site. This is in agreement with the proposals of Milton *et al*., NeuroReport 12: 2561-2566 (2001) which suggested that ERAB 102-105 was the Aβ binding domain.

A further comparison of the Aβ peptide sequence with catalase and ERAB anti-sense sequences showed the presence of Aβ-like sequences within the catalase 400-409 and ERAB 99-108 anti-sense sequences.

Synthetic peptides containing catalase residues 400-409 (CAβBP), ERAB residues 99-108 (EAβBP), AβAS(F) residues 14-23 and AβAS(F) residues 27-36 were all synthesised for analysis. The CAβBP and EAβBP peptides were tested for ability to bind biotinylated Aβ. All peptides were also tested in catalase inhibition and cytotoxicity assays.

Biotinylated Aβ 1-42, Aβ 12-28 and Aβ 25-35 (from SEQ ID NO. 2) were prepared using a LinKit-Biolink kit (ISL, Paignton, UK). ELISA plates were coated with CAβBP (catalase residues 400-409) or EAβBD (ERAB residues 99-108) (1 µg ml⁻¹) in carbonate buffer and unoccupied sites blocked with 5% (w/v) dried milk. Biotinylated peptides (200 pM) were incubated alone, with control peptides or with unlabelled Aβ peptides in PBS (containing 0.1% BSA and 0.05% Tween-20) at 37°C for 4 hours. After washing to remove unbound material, an alkaline phosphatase polymer-streptavidin conjugate (Sigma, Dorset, UK) was added and incubated at 37°C for 2 hours. After washing to remove unbound material, p-nitrophenylphosphate substrate was added and absorbance at 405 nm determined. Affinity constants were determined by incubating catalase or CAβBP coated plates with biotinylated peptides (200 pM) plus Aβ peptides over a range of concentrations (0 - 100 nM) and detection of bound peptides was carried out by ELISA.

SP2/0-Ag-14 mouse myeloma. cells were maintained in RPMI 1640 medium containing 10% fetal calf serum at 37°C in a 5% CO₂ humidified atmosphere. For cytotoxicity experiments 2 x 10⁵ cells were plated in 24 well dishes in 1 ml PBS containing 0.1 % BSA and test peptides (20 µM) for 24 hours. Cell viability was determined by trypan blue dye exclusion with at least 100 cells counted per well (Milton, Biochem. J. 344: 293-296 (1999)).

For catalase activity catalase EC 1.11.1.6 from human erythrocytes (Sigma, Dorset, UK) was used for all incubation experiments. Activity of Catalase (5kU I⁻¹) incubated with test peptides (2 µM) was determined after incubation in 60 mM sodium-potassium phosphate buffer at 37°C in a total volume of 100 µl. After incubation catalase activity was determined by mixing 50 µl sample with 50 µl substrate (6.5 µmol H₂O₂ in phosphate buffer) for 60 secs, adding 100 µl of 32.4 mM ammonium molybdate and measurement of absorbance change at 405 nm. Catalase activity was calculated from a standard curve (0-100kU l⁻¹) using purified human catalase of known activity (Milton, Biochem. J. 344: 293-296 (1999)).

The CAβBP peptide specifically bound biotinylated Aβ 1-42 and Aβ 25-35 but not Aβ 12-28. Binding of the CAβBP peptide to biotinylated Aβ 1-42 was inhibited by fragments of Aβ 1-42 containing residues 31-35. Scatchard analysis of Aβ 1-42 binding was carried out according to Friguet *et al*, J. Imm. Meth.; 77: 305-319 (1985). Data demonstrated a K_{D} = 1.2 ± 0.1 nM (n=5) for Aβ 1-42 binding the CAβBP peptide. The binding specificity of the CAβBP peptide is identical to that for catalase and the binding K_{D} for catalase is comparable at 3.3 nM (Milton, Biochem. J. 344: 293-296 (1999)).

The CAβBP peptide was able to block the inhibition of catalase enzyme activity by Aβ 1-42 and Aβ fragments containing residues 31-35. The CAβBP peptide was also able to block the cytotoxicity of Aβ 1-42 and Aβ fragments containing residues 31-35.

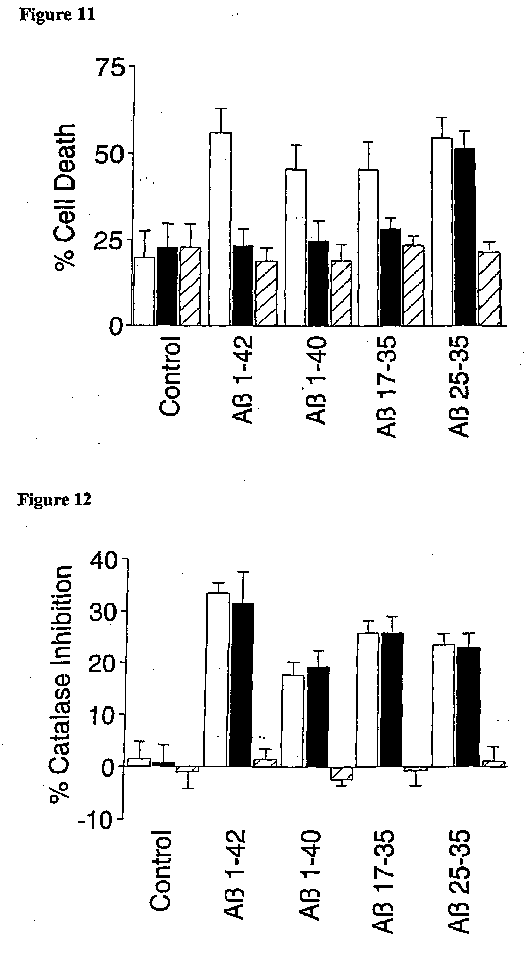

The CAβBP and AβAS(F) 27-36 (from SEQ ID NO. 4) sequences show sequence similarity. The AβAS(F) 27-36 peptide was also able to block the cytotoxicity of Aβ 1-42 and Aβ fragments containing residues 25-35 (Figure 11). The AβAS(F) 27-36 peptide was also able to block the inhibition of catalase enzyme activity by Aβ 1-42 and Aβ fragments containing residues 25-35 (Figure 12).

The EAβBP peptide specifically bound biotinylated Aβ 1-42 and Aβ 12-28 but not Aβ 25-35. Binding of the EAβBP peptide to biotinylated Aβ 1-42 was inhibited byfragments of Aβ 1-42 containing residues 17-24. Scatchard analysis of Aβ 1-42 binding data demonstrated a K_{D} = 107 ± 21 nM (n=5) for Aβ 1-42 binding the EAβBD peptide. The binding specificity of the EAβBD peptide is similar to that for ERAB and the binding K_{D} for ERAS is comparable at 88.3 nM (Oppermann, *et al*., FEBS Lett. 451: 238-242 (1999); Yan, *et al*., Nature 389: 689-695 (1997)).

The EAβBP peptide had no effect on the inhibition of catalase enzyme activity by Aβ 1-42. The EAβBP peptide was able to block the cytotoxicity of Aβ 1-42 and Aβ fragments containing residues 17-35, but not the cytotoxicity of Aβ 25-35, in agreement with a binding specificity for Aβ 17-24.

The EAβBP and AβAS(F) 14-23 sequences show sequence similarity. Like EAβBP, the AβAS(F) 14-23 peptide was able to block the cytotoxicity of Aβ 1-42 and Aβ fragments containing residues 17-35, but not the cytotoxicity of Aβ 25-35 (Figure 11). The AβAS(F) 14-23 peptide had no effect on the inhibition of catalase enzyme activity by Aβ 1-42 and Aβ fragments (Figure 12).

Accordingly, peptides which can bind to the Aβ protein sequence within the Aβ 1-42 region, preferably the Aβ 17-35 region, will be of use. Suitable peptides may be derived from the anti-sense peptides identified herein.

## Claims

1. A phosphorylated Aβ 1-43 protein (SEQ ID No.2), or a phosphorylated fragment thereof of at least 6 amino acids, for use in therapy.

2. A protein according to claim 1, comprising a phosphorylated serine 26 residue.

3. A protein according to claim 1 or claim 2, lacking the amino acid residues indicated as 31-35 in SEQ ID NO. 2.

4. An antibody, raised against a protein according to any of claims 1 to 3, the antibody having no or reduced affinity for the non-phosphorylated form of the protein.

5. An assay for the identification of an agent that binds to Aβ within the region Aβ 1-43, comprising contacting a target agent with a peptide, as defined in any of claims 1 to 3, and determining whether the agent binds to the peptide.

6. A vaccine composition, comprising a protein as defined in any of claims 1 to 3, and a pharmaceutically acceptable diluent or adjuvant.

7. A vaccine according to claim 6, comprising a Aβ 1-43 protein phosphorylated on one or more of residues 8, 10, 26 and 43, and a pharmaceutically acceptable diluent.

8. A compound that blocks the activity of phosphorylated Aβ 1-43 protein.

9. A method for determining whether a patient is at risk from Alzheimer's disease, comprising analysing a sample from the patient that contains Aβ to determine whether Aβ is phosphorylated, where the detection of phosphorylation indicates a risk of Alzheimer's disease.

10. A method according to claim 9, wherein phosphorylation is to be detected within the Aβ 1-43 region.

11. A method according to claim 9 or claim 10, wherein the phosphorylation to be detected is the phosphorylation of a serine amino acid residue.

12. A method according to any of claims 9 to 11, wherein the sample is treated with an antibody that has affinity for Aβ phosphorylated within the Aβ 1-43 region, and has no or reduced affinity for non-phosphorylated Aβ.


SEQUENCE LISTING

<110> Insight Biotechnology Limited

<120> PEPTIDES FOR USE IN THE TREATMENT OF ALZHEIMER'S
      DISEASE

<130> JWJ01146EP

<140> (not yet known)
<141> 2001-11-01

<150> 0026738.5
<151> 2000-11-01

<150> 0026739.3
<151> 2000-11-01

<160> 23

<170> PatentIn Ver. 2.1

<210> 1
<211> 129
<212> DNA
<213> Homo sapiens

<220>
<221> CDS
<222> (1)..(129)

<400> 1
gat gca gaa ttc cga cat gac tca gga tat gaa gtt cat cat caa aaa    48
Asp Ala Glu Phe Arg His Asp Ser Gly Tyr Glu Val His His Gln Lys
  1               5                  10                  15

ttg gtg ttc ttt gca gaa gat gtg ggt tca aac aaa ggt gca atc att    96
Leu Val Phe Phe Ala Glu Asp Val Gly Ser Asn Lys Gly Ala Ile Ile
             20                  25                  30

gga ctc atg gtg ggc ggt gtt gtc ata gcg aca                        129
Gly Leu Met Val Gly Gly Val Val Ile Ala Thr
         35                  40

<210> 2
<211> 43
<212> PRT

<213> Homo sapiens

<400> 2
Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40

<210> 3
<211> 129
<212> DNA
<213> Homo sapiens

<400> 3
ctacgtctta aggctgtact gagtcctata cttcaagtag tagtttttaa ccacaagaaa 60

cgtcttctac acccaagttt gtttccacgt tagtaacctg agtaccaccc gccacaacag 120

tatcgctgt                                                         129

<210> 4
<211> 43
<212> PRT
<213> Artificial Sequence

<220>
<223> Description of Artificial Sequence: Anti-sense
      Peptide

<220>
<221> VARIANT
<222> (5)
<223> X = A or S

<220>
<221> VARIANT
<222> (8)
<223> X = S or R

<220>
<221> VARIANT
<222> (10)

<223> X = I or M

<220>
<221> VARIANT
<222> (12)
<223> X = Q or H

<220>
<221> VARIANT
<222> (17)
<223> X = N, D or E

<220>
<221> VARIANT
<222> (18)
<223> X = H or Q

<220>
<221> VARIANT
<222> (24)
<223> X = H or Q

<220>
<221> VARIANT
<222> (26)
<223> X = S or R

<220>
<221> VARIANT
<222> (34)
<223> X = E, N or D

<220>
<221> VARIANT
<222> (36)
<223> X = H or Q

<220>
<221> VARIANT
<222> (39)
<223> X = H or Q

<220>
<221> UNSURE
<222> (40)
<223> X = H or Q

<220>

<221> VARIANT
<222> (43)
<223> X = C or W

<400> 4
Leu Arg Leu Lys Xaa Val Leu Xaa Pro Xaa Leu Xaa Val Val Val Phe
1               5                   10                  15

Xaa Xaa Lys Lys Arg Leu Leu Xaa Pro Xaa Leu Phe Pro Arg Tyr Tyr
            20                  25                  30

Pro Xaa Tyr Xaa Pro Pro Xaa Xaa Tyr Arg Xaa
        35                  40


<210> 5
<211> 129
<212> DNA
<213> Artificial Sequence

<220>
<223> Description of Artificial Sequence: Synthetic DNA

<400> 5
atctgcttcg aatcgatggt ctgatccata ttcaacatga tgttgtttca acacgaaaaa 60

tgcttcatcc acacctgagt ttttacctgc gataattccg agcatcacgc caccaacgac 120

tatcgctgt                                                         129


<210> 6
<211> 43
<212> PRT
<213> Artificial Sequence

<220>
<223> Description of Artificial Sequence: Anti-sense
      Peptide

<220>
<221> VARIANT
<222> (8)
<223> X = A, R, G or T

<220>
<221> VARIANT
<222> (26)

<223> X = A, R, G or T

<400> 6
Ile Cys Phe Glu Ser Met Val Xaa Ser Ile Phe Asn Met Met Leu Phe
1               5                   10                  15

Gln His Glu Lys Cys Phe Ile His Thr Xaa Val Phe Thr Cys Asp Asn
            20                  25                  30

Ser Glu His His Ala Thr Asn Asp Tyr Arg Cys
        35                  40

<210> 7
<211> 43
<212> PRT
<213> Artificial Sequence

<220>
<223> Description of Artificial Sequence: Anti-sense
      Peptide

<400> 7
Cys Arg Tyr Gln Gln Pro Pro His Tyr Glu Pro Tyr Tyr Arg Pro Phe
1               5                   10                  15

Leu Ser Pro His Leu Leu Arg Lys Lys His Asn Phe Val Val Val Gln
            20                  25                  30

Leu Ile Pro Ser Leu Val Ala Lys Leu Arg Leu
        35                  40

<210> 8
<211> 43
<212> PRT
<213> Artificial Sequence

<220>
<223> Description of Artificial Sequence: Anti-sense
      Peptide

<220>
<221> VARIANT
<222> (18)
<223> X = A, R, G or T

<220>

<221> VARIANT
<222> (36)
<223> X = A, R, G or T

<400> 8
Cys Arg Tyr Asp Asn Thr Ala His His Glu Ser Asn Asp Cys Thr Phe
 1               5                  10                  15

Val Xaa Thr His Ile Phe Cys Lys Glu His Gln Phe Leu Met Met Asn
            20                  25                  30

Phe Ile Ser Xaa Val Met Ser Glu Phe Cys Ile
        35                  40

<210> 9
<211> 43
<212> PRT
<213> Artificial Sequence

<220>
<223> Description of Artificial Sequence: Anti-sense
      Peptide

<220>
<221> VARIANT
<222> (1)
<223> X = L, I or V

<220>
<221> VARIANT
<222> (5)
<223> X = A, S, P or T

<220>
<221> VARIANT
<222> (7)
<223> X = L, I or V

<220>
<221> VARIANT
<222> (15)
<223> X = L or V

<220>
<221> VARIANT
<222> (23)
<223> X = L, I or V

<220>
<221> VARIANT
<222> (27)
<223> X = L, I or V

<220>
<221> VARIANT
<222> (35)
<223> X = Y or H

<400> 9
Xaa Arg Leu Lys Xaa Val Xaa Arg Pro Ile Leu His Val Val Xaa Phe
1               5                   10                  15

Glu His Lys Lys Arg Leu Xaa His Pro Arg Xaa Phe Pro Arg Tyr Tyr
            20                  25                  30

Pro Glu Xaa His Pro Pro His His Tyr Arg Cys
        35                  40


<210> 10
<211> 43
<212> PRT
<213> Artificial Sequence

<220>
<223> Description of Artificial Sequence: Anti-sense
      Peptide

<220>
<221> VARIANT
<222> (9)
<223> X = M, Y or H

<220>
<221> VARIANT
<222> (17)
<223> X = N, L, V or I

<220>
<221> VARIANT
<222> (21)
<223> X = D, L, I or V

<220>
<221> VARIANT

<222> (29)
<223> X = Q, V or L

<220>
<221> VARIANT
<222> (37)
<223> X = D, L, V or I

<220>
<221> VARIANT
<222> (39)
<223> X = R, A, S, P or T

<220>
<221> VARIANT
<222> (43)
<223> X = D, L, I or V

<400> 10
Cys Arg Tyr His His Pro Pro His Xaa Glu Pro Tyr Tyr Arg Pro Phe
1               5                   10                  15

Xaa Arg Pro His Xaa Leu Arg Lys Lys His Glu Phe Xaa Val Val His
            20                  25                  30

Leu Ile Pro Arg Xaa Val Xaa Lys Leu Arg Xaa
        35                  40

<210> 11
<400> 11
000

<210> 12
<400> 12
000

<210> 13
<211> 8
<212> PRT
<213> Homo sapiens

<400> 13
Lys Glu Leu Arg His Pro Asn Ile
1               5

<210> 14

<211> 5
<212> PRT
<213> Homo sapiens

<400> 14
Pro Pro Gly Gln Tyr
1               5

<210> 15
<211> 28
<212> PRT
<213> Homo sapiens

<400> 15
Val Lys Ser Tyr Leu Tyr Gln Ile Leu Gln Gly Ile Val Phe Cys His
1               5                   10                  15

Ser Arg Arg Val Leu His Arg Asp Leu Lys Pro Gln
            20                  25

<210> 16
<211> 10
<212> PRT
<213> Homo sapiens

<400> 16
Pro Glu Val Glu Ser Leu Gln Asp Tyr Lys
1               5                   10

<210> 17
<211> 29
<212> PRT
<213> Homo sapiens

<400> 17
Lys Tyr Glu Glu Met Tyr Pro Pro Glu Ile Gly Asp Phe Ala Phe Val
1               5                   10                  15

Thr Asp Asn Thr Tyr Thr Lys His Gln Ile Arg Gln Met
            20                  25

<210> 18
<211> 7
<212> PRT

<213> Homo sapiens

<400> 18
Pro Ile Leu Phe Pro Ser Phe
1               5

<210> 19
<211> 7
<212> PRT
<213> Homo sapiens

<400> 19
Ile Gln Val Met Thr Phe Asn
1               5

<210> 20
<211> 13
<212> PRT
<213> Homo sapiens

<400> 20
Pro Asn Tyr Tyr Pro Asn Ser Phe Gly Ala Pro Glu Gln
1               5                   10

<210> 21
<211> 9
<212> PRT
<213> Homo sapiens

<400> 21
Tyr Asn Leu Lys Lys Gly Gln Thr His
1               5

<210> 22
<211> 10
<212> PRT
<213> Homo sapiens

<400> 22
Pro Lys Thr Pro Lys Lys Ala Lys Lys Leu
1               5                   10

<210> 23
<211> 43
<212> PRT
<213> Artificial Sequence

<220>
<221> VARIANT
<222> (2)
<223> X = R, G or S

<220>
<221> VARIANT
<222> (5)
<223> X = A, P or T

<220>
<221> VARIANT
<222> (8)
<223> X = A, R, G or T

<220>
<221> VARIANT
<222> (9)
<223> X = A, P or T

<220>
<221> VARIANT
<222> (12)
<223> X = D, H or Y

<220>
<221> VARIANT
<222> (17)
<223> X = E or K

<220>
<221> VARIANT
<222> (18)
<223> X = N, D or Y

<220>
<221> VARIANT
<222> (21)
<223> X = R, G or S

<220>
<221> VARIANT
<222> (24)

<223> X = N, D or Y

<220>
<221> VARIANT
<222> (25)
<223> X = A, P or S

<220>
<221> VARIANT
<222> (26)
<223> X = A, R, G or T

<220>
<221> VARIANT
<222> (29)
<223> X = A, P or S

<220>
<221> VARIANT
<222> (30)
<223> X = R, G or S

<220>
<221> VARIANT
<222> (31)
<223> X = N or Y

<220>
<221> VARIANT
<222> (32)
<223> X = D or Y

<220>
<221> VARIANT
<222> (33)
<223> X = A, P or T

<220>
<221> VARIANT
<222> (34)
<223> X = Q or K

<220>
<221> VARIANT
<222> (36)
<223> X = N, D or Y

<220>

<221> VARIANT
<222> (37)
<223> X = P, S or T

<220>
<221> VARIANT
<222> (38)
<223> X = A, P or S

<220>
<221> VARIANT
<222> (39)
<223> X = D, H or Y

<220>
<221> VARIANT
<222> (40)
<223> X = N, H or Y

<220>
<221> VARIANT
<222> (41)
<223> X = N or D

<220>
<221> VARIANT
<222> (42)
<223> X = C, G or S

<220>
<221> VARIANT
<222> (43)
<223> X = R, G or S

<220>
<223> Description of Artificial Sequence: Anti-sense
      Peptide

<400> 23
Val Xaa Leu Lys Xaa Val Ile Xaa Xaa Val Leu Asp Val Val Leu Leu
  1               5                  10                  15

Xaa Xaa Lys Glu Xaa Leu Val Xaa Xaa Xaa Ile Leu Xaa Xaa Xaa Xaa
             20                  25                  30

Xaa Xaa His Xaa Xaa Xaa Xaa Xaa Xaa Xaa Xaa
         35                  40